# EUROPEAN PATENT APPLICATION

(11) **EP 4 074 311 A2**
(43) Date of publication of application: **19.10.2022**
(21) Application number: 20900141.1
(22) Date of filing: 11.12.2020
(51) Int. Cl.: A61K 31/28, A61K 31/555, A61K 9/00, A61P 17/02, A61K 8/58, A61Q 19/00, A61L 15/44, A61L 15/60, A61F 13/00

(54) **COMPOSITION FOR WOUND HEALING, CONTAINING METAL-ORGANIC FRAMEWORK**

(30) Priority: 13.12.2019 KR 20190167194; 10.12.2020 KR 20200172690
(71) Applicant: Labincube Co., Ltd., Chungcheongbuk-do 28116 (KR)
(72) Inventor: HEO, Chan Yeong, Yongin-si, Gyeonggi-do 16850 (KR); NAM, Sun-Young, Anyang-si, Gyeonggi-do 14027 (KR); JEONG, Jae Heon, Seongnam-si, Gyeonggi-do 13607 (KR); CHOI, Kyungmin, Seoul 08097 (KR); SHIN, Dong-Sik, Seoul 04180 (KR); RYU, Un Jin, Seoul 04310 (KR); KIM, Seongsoo, Wonju-si, Gangwon-do 26406 (KR); KIM, Wonil, Gumi-si, Gyeongsangbuk-do 39372 (KR); PARK, Jaejin, Gumi-si, Gyeongsangbuk-do 39345 (KR); SEO, Mingi, Chilgok-gun, Gyeongsangbuk-do 39904 (KR)
(74) Representative: Beck & Rössig European Patent Attorneys
(86) International application number: PCT/KR2020/018159
(87) International publication number: WO 2021/118292

(57) **Abstract**

The present invention relates to: a composition for wound healing, containing a metal-organic framework (MOF)as an active ingredient; and a wound dressing material comprising a hydrophilic superabsorbent polymer and the MOF, and the composition and the wound dressing material have a wound healing ability, specifically, the excellent effect of healing a wound by promoting fibroblast movement, do not significantly differ from a control group with respect to cytotoxicity, thereby exhibiting excellent safety when applied to a wound site, and have the excellent effect of having a wound recovery rate high enough to be checked with the naked eye over time.

## Description

### Technical Field

In the specification, a composition for wound healing, which includes a metal-organic framework (MOF) as an active ingredient, and a wound dressing material comprising a hydrophilic superabsorbent polymer and the MOF are disclosed.

### National R&D Project Supporting the Invention

This study was supported by the technology development project No. S2737226 of the Ministry of SMEs and Startups.

### Background Art

Skin consists of three major layers: the epidermis, the dermis and the subcutaneous fat. Each layer of skin tissue consists of various cells responsible for several functions of the skin and materials surrounding the cells. When the skin is damaged, the damaged site is invaded by bacteria and external toxins, and the skin defensive function against them causes inflammation.

Wound healing is a tissue recovery process by a tissue response to damaged skin, and a complicated biological process that includes chemotaxis, cell differentiation and replication, lipoprotein synthesis and angiogenesis and the like.

In the tissue recovery process for damaged skin, cell proliferation works with cell migration to restore the damaged skin site. That is, in order to repair damaged skin tissue, a process of activating skin cell generation and migrating newly generated cells to the damaged site and forming new skin tissue is undergone. Therefore, for wound healing, it is necessary to use a drug that is responsible for promoting the proliferation of skin cells and controlling the migration of the generated skin cells to the damaged skin site.

Meanwhile, in order to speed up wound healing and minimize various secondary side effects, wound treatment using an appropriate covering material or dressing is essential. As conventional treatment methods for skin wounds such as burns, trauma, wounds and bedsores, wounds were treated by applying an antiseptic to a wound, covering the wounded site with an absorbent material such as gauze or cotton to absorb an exudate generated from the wound and dry the wound site. However, recent studies revealed that maintaining an appropriate moist environment is rather effective for wound treatment, and various types and materials of wound dressing materials have been developed and marketed.

Under this background, the present inventors conducted research on a material that can show an excellent wound healing effect and be prepared as a wound dressing material, and thus the present invention was completed.

### Related Art Documents

### Patent Documents

(Patent Document 1) KR 10-2019-0072333 A
(Patent Document 2) KR 10-2019-0083593 A
(Patent Document 3) KR 10-2010-0023073 A

### Disclosure

### Technical Problem

The present invention is directed to providing a composition for wound healing, which includes a metal-organic framework (MOF) as an active ingredient.

The present invention is also directed to providing a wound dressing material which comprises a hydrophilic superabsorbent polymer and an MOF.

### Technical Solution

In one aspect, the present invention provides a composition for wound healing, which includes a metal-organic framework (MOF) as an active ingredient.

In another aspect, the present invention provides a wound dressing material which comprises a hydrophilic superabsorbent polymer and an MOF.

### Advantageous Effects

The present invention relates to a composition including a metal-organic framework (MOF), which has an excellent wound healing ability, specifically, an excellent effect of healing wounds by promoting fibroblast migration, excellent safety when applied to the wound site because there is no significant difference from a control in terms of cytotoxicity, and an excellent wound recovery rate that can be visually confirmed as time passes in the case of the wound dressing material comprising a hydrophilic superabsorbent polymer and the MOF.

### Description of Drawings

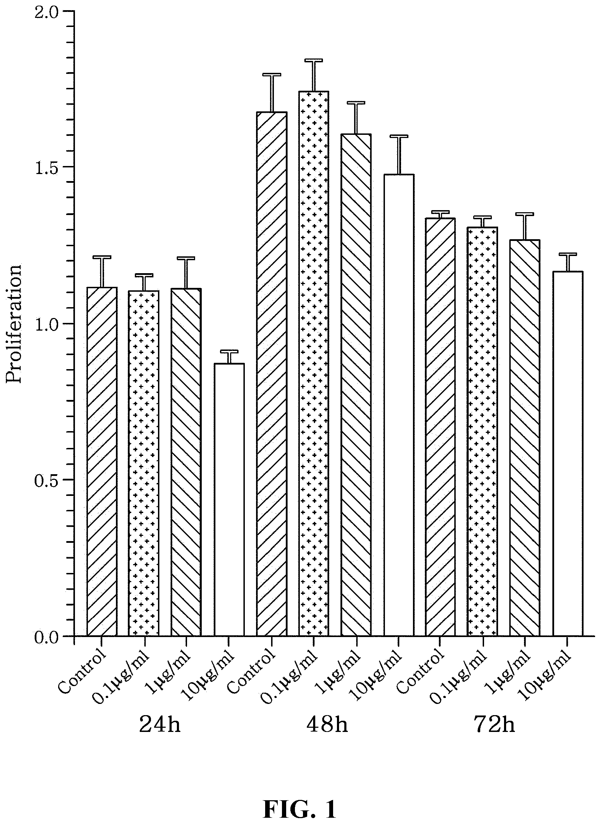

FIG. 1 is a graph showing a cytotoxicity test result for fibroblasts treated with a metal-organic framework (MOF) according to one embodiment of the present invention. The x axis represents concentrations of an MOF treated for 24 h, 48 h and 72 h (Control; 0.1, 1 and 10 µg/ml), and the y axis represents fibroblast viability.

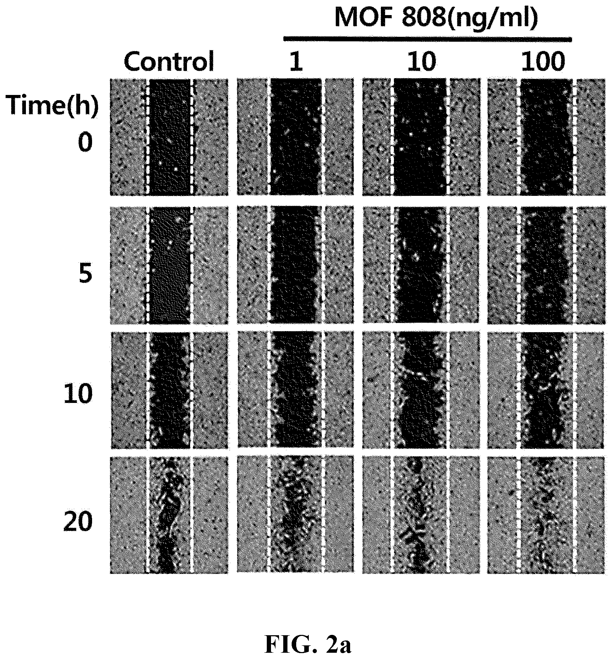

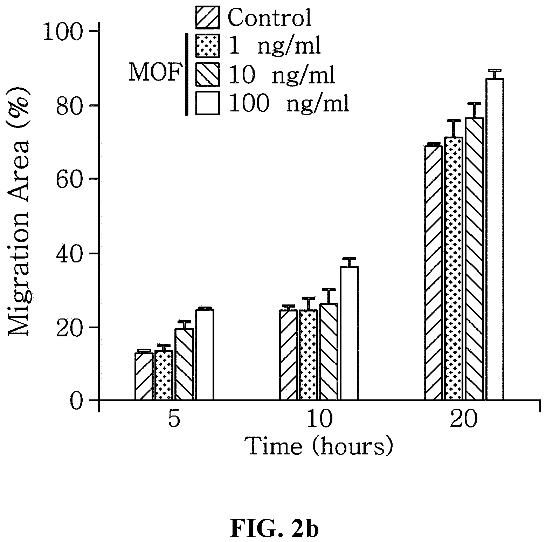

FIGS. 2A and 2B show the wound healing ability of an MOF when mouse fibroblasts with a scratch were treated with the MOF according to one embodiment of the present invention, in which FIG. 2A is a set of images showing the wound healing ability by fibroblast migration over time, and FIG. 2B is a graph comparing a migration area of the fibroblasts. In the graph of FIG. 2B, the x axis represents the concentrations of the MOF treated for 5, 10 and 20 hours (Control; 1, 10 and 100 ng/ml), the y axis represents the migration area (%) of fibroblasts, and the higher the migration area of the fibroblasts, the better the wound healing ability.

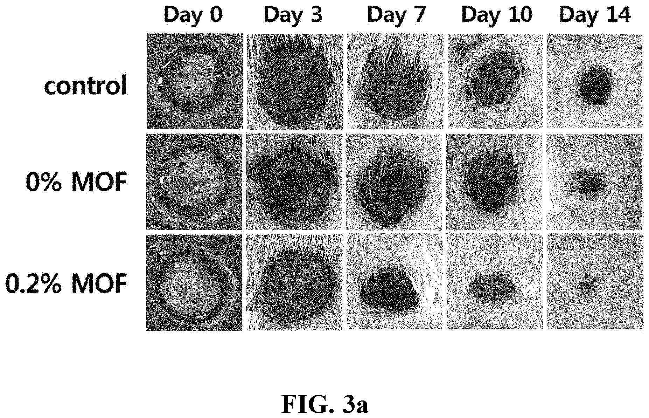

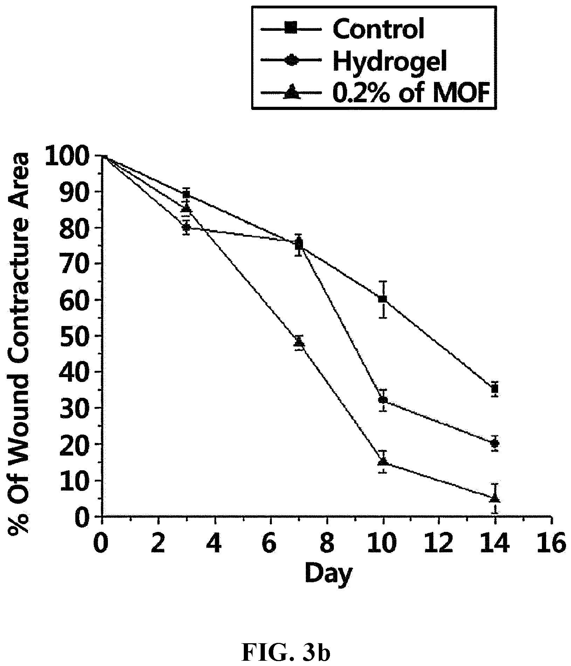

FIGS. 3A and 3B show a set of images of the wounded dorsal areas of rats on day 0, 3, 7, 10 and 14 after dressing with an MOF-dispersed hydrogel (Control; content: 0 wt%, 0.2 wt%) according to one embodiment of the present invention (FIG. 3A) and a graph showing the comparison of wound contracture areas (FIG. 3B). The x axis of the graph of FIG. 3B represents the time elapsed after dressing (day), the y axis represents wound contracture area (%), and the lower the wound contracture area, the better the wound healing ability.

### Modes of the Invention

Hereinafter, the present invention will be described in detail.

In the present invention, the term "metal-organic framework (MOF)" refers to a porous material in which metal clusters and organic linkers (or organic bridging ligands) are connected by a coordinate bond to form a three-dimensional structure, and may form various MOFs according to the choice of a metal ion and an organic ligand. The MOF is porous, which means that there is an empty space in its structure, and depending on the types and binding types of metal ions and organic ligands constituting an MOF, the pore size, porosity, three-dimensional structure and surface area may be designed in various ways. Due to this porosity, the MOF has a very large surface area and an open pore structure, so it is possible to move a large amount of molecules or solvents, compared to other porous materials known in the art, and there is the advantage of maximizing efficiency due to many active sites when used as a catalyst or gas storing body. In addition, the MOF is not easily deformed at a high temperature and has a rigid backbone, so it has excellent chemical stability and excellent thermal stability.

In one aspect, the present invention provides a composition for wound healing, which includes an MOF as an active ingredient.

In one embodiment of the present invention, the MOF may include one or more selected from the group consisting of Al and Zr, and one or more organic ligands selected from the group consisting of 4,4'-biphenyldicarboxylic acid, benzene-1,4-dicarboxylic acid, 9,10-anthracenedicarboxylic acid, biphenyl-3,3,5,5'-tetracarboxylic acid, biphenyl-3,4',5-tricarboxylic acid, 5-bromoisophthalic acid, 5-cyano-1,3-benzenedicarboxylic acid, 2,2-diamino-4,4'-stilbenedicarboxylic acid, 2,5-diaminoterephthalic acid, 1,1,2,2-tetra(4-carboxylphenyl)ethylene, 2,5-dihydroxyterephthalic acid, 2,2-dinitro-4,4-stilbenedicarboxylic acid, 5-ethynyl-1,3-benzenedicarboxylic acid, 2-hydroxyterephthalic acid, 2,6-naphthalenedicarboxylic acid, 1,2,4,5-tetrakis(4-carboxyphenyl)benzene, 4,4,4"-s-triazine-2,4,6-triyl-tribenzoic acid, 1,3,5-tricarboxybenzene, 1,4,7,10-tetraazacyclododecane-N,N',N",N'"-tetraacetic acid, 1,3,5-tris(4-carboxy1,1'-niphenyl- 4-yl)benzene, 1,3,5-tris(4-carboxyphenyl)benzene, and 1,3,5-triscarboxyphenylethynylbenzene. Specifically, the MOF may be Zr₆O₄(OH)₄(BTC)₂(HCOO)₆ (MOF-808), Zr₆O₄(OH)₄(fumarate)₆ (MOF-801), Zr₆O₆(BDC)₆, Zr₆O₆(NH-₂-BDC)₆, Zr₆O₆(OH)₄(BPDC)₆, Al₃O(OH)(H₂O)₂(BDC-NH₂)₃ or Al(OH)(BDC), and more specifically, Zr₆O₄(OH)₄(BTC)₂(HCOO)₆ (MOF-808), and may be used to ensure stability when treating a wounded skin site, and to heal the wound. Further, the type of MOF is not limited as long as it is an MOF that has safety, stability and wound healing ability to be used as an active ingredient of the wound dressing material.

In one embodiment of the present invention, the MOF may promote skin cell migration. In addition, in one embodiment of the present invention, the composition including the MOF may promote skin cell migration. In the wound healing process, cell proliferation and migration are important, and the MOF may promote cell migration, thereby enhancing a wound healing effect. Specifically, the skin cells may be skin fibroblasts, but the present invention is not limited thereto. According to one embodiment of the present invention, when fibroblasts with scratches are treated with the MOF, it was confirmed that, as the time passes and the MOF concentration increases, the fibroblasts migrate faster than the control, and it was confirmed that the composition including the MOF has an excellent wound healing effect according to the promotion of fibroblast migration (Experimental Example 2 and FIGS. 2A and 2B).

In one embodiment of the present invention, the concentration of the MOF may be 0.001 to 10 µg/ml. Specifically, the concentration of the MOF may be 0.001 µg/ml or more, 0.005 µg/ml or more, 0.01 µg/ml or more, 0.02 µg/ml or more, 0.03 µg/ml or more, 0.04 µg/ml or more, 0.05 µg/ml or more, 0.06 µg/ml or more, 0.07 µg/ml or more, 0.08 µg/ml or more, 0.09 µg/ml or more, 0.1 µg/ml or more, 0.11 µg/ml or more, 0.12 µg/ml or more, 0.13 µg/ml or more, 0.14 µg/ml or more, 0.15 µg/ml or more, 0.16 µg/ml or more, 0.17 µg/ml or more, 0.18 µg/ml or more, 0.19 µg/ml or more, 0.2 µg/ml or more, 0.3 µg/ml or more, 0.4 µg/ml or more, 0.5 µg/ml or more, 0.6 µg/ml or more, 0.7 µg/ml or more, 0.8 µg/ml or more, 0.9 µg/ml or more, 1 µg/ml or more, 2 µg/ml or more, 4 µg/ml or more, 6 µg/ml or more, 8 µg/ml or more or 10 µg/ml or more, and the concentration of the MOF may be 10 µg/ml or less, 8 µg/ml or less, 6 µg/ml or less, 4 µg/ml or less, 2 µg/ml or less, 1 µg/ml or less, 0.9 µg/ml or less, 0.8 µg/ml or less, 0.7 µg/ml or less, 0.6 µg/ml or less, 0.5 µg/ml or less, 0.4 µg/ml or less, 0.3 µg/ml or less, 0.2 µg/ml or less, 0.19 µg/ml or less, 0.18 µg/ml or less, 0.17 µg/ml or less, 0.16 µg/ml or less, 0.15 µg/ml or less, 0.14 µg/ml or less, 0.13 µg/ml or less, 0.12 µg/ml or less, 0.11 µg/ml or less, 0.1 µg/ml or less, 0.09 µg/ml or less, 0.08 µg/ml or less, 0.07 µg/ml or less, 0.06 µg/ml or less, 0.05 µg/ml or less, 0.04 µg/ml or less, 0.03 µg/ml or less, 0.02 µg/ml or less, 0.01 µg/ml or less, 0.005 µg/ml or less, or 0.001 µg/ml or less. The MOF may be used to ensure safety against cytotoxicity when treating the wounded site of the skin, and may be used for wound healing. The concentration of the MOF is not limited as long as the MOF is included as an active ingredient of the wound dressing material to have safety, stability and wound healing ability.

In one embodiment of the present invention, the MOF may be included at 0.01 to 10 wt% based on the total weight of the composition. Specifically, the MOF may be included at 0.01 wt% or more, 0.05 wt% or more, 0.1 wt% or more, 0.11 wt% or more, 0.12 wt% or more, 0.13 wt% or more, 0.14 wt% or more, 0.15 wt% or more, 0.16 wt% or more, 0.17 wt% or more, 0.18 wt% or more, 0.19 wt% or more, 0.2 wt% or more, 0.25 wt% or more, 0.3 wt% or more, 0.35 wt% or more, 0.4 wt% or more, 0.45 wt% or more, 0.5 wt% or more, 0.55 wt% or more, 0.6 wt% or more, 0.65 wt% or more, 0.7 wt% or more, 0.71 wt% or more, 0.72 wt% or more, 0.73 wt% or more, 0.74 wt% or more, 0.75 wt% or more, 0.76 wt% or more, 0.77 wt% or more, 0.78 wt% or more, 0.79 wt% or more, 0.8 wt% or more, 0.85 wt% or more, 0.9 wt% or more, 0.95 wt% or more, 1 wt% or more, 5 wt% or more, or 10 wt% or more based on the total weight of the composition, and the MOF may be included at 10 wt% or less, 5 wt% or less, 1 wt% or less, 0.95 wt% or less, 0.9 wt% or less, 0.89 wt% or less, 0.88 wt% or less, 0.87 wt% or less, 0.86 wt% or less, 0.85 wt% or less, 0.84 wt% or less, 0.83 wt% or less, 0.82 wt% or less, 0.81 wt% or less, 0.8 wt% or less, 0.75 wt% or less, 0.7 wt% or less, 0.65 wt% or less, 0.6 wt% or less, 0.55 wt% or less, 0.5 wt% or less, 0.45 wt% or less, 0.4 wt% or less, 0.35 wt% or less, 0.3 wt% or less, 0.29 wt% or less, 0.28 wt% or less, 0.27 wt% or less, 0.26 wt% or less, 0.25 wt% or less, 0.24 wt% or less, 0.23 wt% or less, 0.22 wt% or less, 0.21 wt% or less, 0.2 wt% or less, 0.15 wt% or less, 0.1 wt% or less, 0.04 wt% or less, or 0.01 wt% based on the total weight of the composition. The MOF may be used to ensure safety against cytotoxicity when treating a wounded site of the skin, and may be used for wound healing. The concentration of the MOF is not limited as long as the MOF is included as an active ingredient of the wound dressing material to have safety, stability and wound healing ability.

In one embodiment of the present invention, wounds encompass damaged body parts and refer to a state in which the body is damaged, and are also called injuries. The wound may be one or more selected from the group consisting of an injury, a non-healing traumatic wound, destruction of tissue by irradiation, an abrasion, a laceration, an avulsion, a penetrating injury, a bullet wound, a cut, a burn, frostbite, a sore, dry skin, keratoderma, a crack, chapped skin, dermatitis, a surgical or vascular disease wound, a bruise, a corneal wound, a bedsore, an abscess, a chronic ulcer, an operative suture site, a spinal injury, a gynecological wound, a chemical wound and acne, but the present invention is not limited thereto. In addition, in one embodiment of the present invention, a wound and an injury may be used interchangeably. According to one embodiment of the present invention, in the case of dressing the dorsal area of a rat having a full-layer skin wound with a dressing material including the MOF according to one embodiment of the present invention, the wound healed over time, it was confirmed that the composition including the MOF according to one embodiment of the present invention has a wound healing ability, and thus can be used as a high-functional dressing material for wound treatment or healing (Experimental Example 3 and FIG. 3).

In one embodiment of the present invention, the composition may be a composition for external application to the skin, and may be a liquid, a cream, a paste or a solid.

In one embodiment of the present invention, the composition may be a pharmaceutical composition or cosmetic composition.

In one embodiment of the present invention, the pharmaceutical composition may be a composition for wound healing or treatment.

The pharmaceutical composition may be provided in all formulations suitable for topical administration. For example, pharmaceutical composition may be a dermal solution for external use, a suspension, an emulsion, a gel, a patch or a spray, but the present invention is not limited thereto. The formulation may be easily prepared by a conventional method known in the art, and a surfactant, an excipient, a hydrating agent, an emulsifier, a suspending agent, a salt or buffer for controlling osmotic pressure, a colorant, a spice, a stabilizer, a preservative, a preserving agent, or other commonly used adjuvants may be used.

The pharmaceutical composition may further include a pharmaceutically acceptable carrier, excipient or diluent as needed in addition to the above ingredients. The pharmaceutically acceptable carrier, excipient or diluent is not limited as long as it does not impair the effect of the present invention, and may include, for example, a filler, an extender, a binder, a wetting agent, a disintegrant, a surfactant, a lubricant, a sweetener, a fragrance or a preservative.

Representative examples of the pharmaceutically acceptable carriers, excipients or diluents may include lactose, dextrose, sucrose, sorbitol, mannitol, xylitol, maltitol, starch, gelatin, glycerin, acacia gum, alginate, calcium phosphate, calcium carbonate, calcium silicate, cellulose, methyl cellulose, microcrystalline cellulose, polyvinyl pyrrolidone, water, methylhydroxybenzoate, propylhydroxybenzoate, talc, magnesium stearate, mineral oil, propylene glycol, polyethylene glycol, vegetable oil, injectable ester, Witepsol, Macrogol, Tween 61, cacao butter, and laurinum.

In addition, in one embodiment of the present invention, when the MOF is used as a quasi-drug, one or more active ingredients exhibiting the same or similar function may be additionally contained. For example, the MOF may include a known wound healing component. The additional component may be included at 0.0001 wt% to 10 wt% with respect to the total weight of the composition, and the content range may be adjusted by a requirement such as skin safety, or ease of formulation of the MOF according to one embodiment of the present invention.

The active ingredient of the pharmaceutical composition according to one embodiment of the present invention may vary according to the age, sex, weight, a pathological condition and its severity, an administration route or the judgement of a prescriber. A dosage based on such a factor is determined at a level of one of ordinary skill in the art, and a daily dose may be, for example, 0.1 mg/kg/day to 5000 mg/kg/day, and more specifically, 50 mg/kg/day to 500 mg/kg/day, but the present invention is not limited thereto.

In one embodiment of the present invention, the cosmetic material composition may be a composition for wound relief or recovery.

The cosmetic material composition may be provided in all formulations suitable for topical application. For example, the cosmetic material composition may be provided in the form of a solution, an emulsion obtained by dispersing an oil phase in a water phase, an emulsion obtained by dispersing a water phase in an oil phase, a suspension, a solid, a gel, a powder, a paste, a foam or an aerosol. The composition of such a formulation may be prepared according to a conventional method known in the art.

The cosmetic material composition may contain other components within a range that does not impair the main effect, specifically, components that can give a synergistic effect to the main effect, in addition to the above-described material. The cosmetic material composition according to one embodiment of the present invention may include a material selected from the group consisting of a vitamin, a polymer peptide, a polymer polysaccharide and a sphingolipid. In addition, the cosmetic material composition according to one embodiment of the present invention may include a moisturizer, an emollient, a surfactant, a UV absorbent, a preservative, a bactericide, an antioxidant, a pH adjustor, organic and inorganic pigments, a fragrance, a cooling agent or a deodorant. The blending amount of the component can be easily selected by those of ordinary skill in the art within the range that does not impair the purpose and effect of the present invention, and the blending amount may be 0.01 to 5 wt%, and specifically 0.01 to 3 wt% based on the total weight of the composition.

In one embodiment of the present invention, the composition may be a quasi-drug composition. In addition, specifically, in one embodiment of the present invention, the composition may be a quasi-drug composition for wound healing, which includes an MOF as an active ingredient.

The quasi-drug composition may further include a pharmaceutically acceptable carrier, an excipient or a diluent as needed, in addition to the above-described component. The pharmaceutically acceptable carrier, excipient or diluent is not limited as long as it does not impair the effect of the present invention, and may include, for example, a filler, an extender, a binder, a wetting agent, a disintegrant, a surfactant, a lubricant, a sweetener, a fragrance or a preservative.

Representative examples of the pharmaceutically acceptable carriers, excipients or diluents may include lactose, dextrose, sucrose, sorbitol, mannitol, xylitol, maltitol, starch, gelatin, glycerin, acacia gum, alginate, calcium phosphate, calcium carbonate, calcium silicate, cellulose, methyl cellulose, microcrystalline cellulose, polyvinyl pyrrolidone, water, methylhydroxybenzoate, propylhydroxybenzoate, talc, magnesium stearate, mineral oil, propylene glycol, polyethylene glycol, vegetable oil, injectable ester, Witepsol, Macrogol, Tween 61, cacao butter, and laurinum.

In addition, when the MOF according to one embodiment of the present invention is used as a quasi-drug, one or more active ingredients exhibiting the same or similar function may be additionally contained. For example, the MOF may include a known wound healing component. The additional component may be included at 0.0001 wt% to 10 wt% with respect to the total weight of the composition, and the content range may be adjusted by a requirement such as skin safety, or ease of formulation of the MOF according to one embodiment of the present invention.

The quasi-drug composition according to one embodiment of the present invention may be a disinfectant cleanser, a shower foam, an ointment, a wet tissue, a coating agent or the like, but the present invention is not limited thereto. The formulation method, dose, usage method and components of the quasi-drug may be suitably selected by a conventional method known in the art.

In another aspect, the present invention may provide a wound dressing material comprising a hydrophilic superabsorbent polymer and an MOF. The wound dressing material may be for wound healing. The description of the MOF and wounds is the same as above.

In one embodiment of the present invention, the hydrophilic superabsorbent polymer refers to a polymer having hydrophilicity among super absorbent polymers (SAPs) having high absorption ability. The superabsorbent polymer may be generally obtained by insolubilizing a water-soluble polymer such as starch, polyacrylic acid, polyvinyl alcohol or polyethylene oxide in water by crosslinking, and may absorb hundreds of times more water in the molecule. The superabsorbent polymer may be, specifically, polyacrylic acid, polyvinyl alcohol, gelatin, alginate or chitosan, but the present invention is not limited thereto. In addition, the hydrophilic superabsorbent polymer may be one or more selected from the group consisting of a hydrogel, hyaluronic acid, polyvinyl pyrrolidone, carboxymethyl cellulose, hydroxy ethyl cellulose, xanthan gum and sodium hyaluronate, and specifically, a hydrogel. However, the MOF according to one embodiment of the present invention may be dispersed, and when dressing the wounded area of the skin, safety is ensured and the wound healing effect is shown, and the type of hydrophilic superabsorbent polymer is not limited as long as it has safety, stability and a wound healing ability to be included as an active ingredient of a wound dressing material.

In one embodiment of the present invention, when the dorsal area of a rat having a full-layer skin wound is dressed with a wound dressing material including a hydrogel in which an MOF is dispersed according to one embodiment of the present invention, the wound healed over time, and it was confirmed that the wound dressing material including a hydrophilic superabsorbent polymer including the MOF according to one embodiment of the present invention has an excellent injury or wound healing effect (Experimental Example 3 and FIG. 3).

In still another aspect, the present invention provides a method for wound healing, which includes administering or applying a composition including an MOF to a subject in need of wound healing. In one embodiment of the present invention, the administration or application in this method may be performed by an administration or application method and an administration or application dose, which are disclosed in the specification.

In yet another aspect, the present invention provides a use of an MOF for preparing a pharmaceutical composition for wound healing.

In yet another aspect, the present invention provides a use of an MOF for preparing a cosmetic material composition for wound healing.

In yet another aspect, the present invention provides a use of an MOF for preparing a quasi-drug composition for wound healing.

In yet another aspect, the present invention provides a use of a composition including an MOF for wound healing.

In yet another embodiment, the present invention provides a composition including an MOF for wound healing.

In yet another aspect, the present invention provides a method for wound healing, which includes: applying a wound dressing material comprising a hydrophilic superabsorbent polymer and an MOF to a subject in need of wound healing. In one embodiment of the present invention, the application in this method may be performed by the dressing method and dressing dose, disclosed in the specification.

In yet another aspect, the present invention provides a use of a wound dressing material comprising a hydrophilic superabsorbent polymer and an MOF for preparing a wound dressing material.

In yet another aspect, the present invention provides a use of a wound dressing material comprising a hydrophilic superabsorbent polymer and an MOF for wound healing.

In yet another aspect, the present invention provides a composition, which includes a wound dressing material comprising a hydrophilic superabsorbent polymer and an MOF for wound healing.

Hereinafter, the configuration and effects of the present invention will be described in further detail with reference to examples and experimental examples. However, the following examples and experimental examples are merely provided for the purpose of illustration to help the understanding of the present invention, and the scope of the present invention is not limited thereto.

### Example 1 Preparation of MOF-808 as MOF

To prepare MOF-808 as an MOF included as an active ingredient for a wound healing composition and a wound dressing material, the following process was performed.

Specifically, 0.485 g (1.5 mmol) of ZrOCl·8H₂O as a metal part was dissolved in 11.25 mL of *N,N*'-dimethylformamide (DMF) in a 50-mL glass vial, and 22.5 mL of formic acid was additionally mixed. Here, after mixing11.25 mL of a DMF solution in which 0.105 g (0.5 mmol) of trimesic acid was mixed, a reaction was carried out at 130 °C for 2 days. Afterward, the resulting product was washed with distilled water and acetone, transferred to distilled water and freeze-dried, thereby preparing MOF-808.

### Example 2 Preparation of wound dressing material

To prepare a wound dressing material containing MOF-808, which is the MOF prepared in Example 1, the following process was performed.

0.1 to 10 wt% of a hydrogel (Sigma-Aldrich, poly(acrylic acid) 306223) as a hydrophilic superabsorbent polymer was dissolved in ultrapure water according to a required viscosity, based on the total weight (500 ml). 0.1 wt% of the MOF-808 prepared in Example 1 was added to the completely-dissolved polymer solution and dispersed using a homogenizer (CAT, X1740) at 2,500 to 10,000 rpm for 1 hour, and the resulting solution was transferred to a mechanical stirrer (Daihan Scientific, HT-120DX), and stirred at 500 to 1,000 rpm for 1 hour while 0.1 wt% of triethanolamine (KPX) as a pH adjuster was dropped, thereby preparing a hydrogel including MOF-808.

### Experimental Example 1 Confirmation of cytotoxicity of MOF

To confirm the degree of toxicity against cells and the suitability as an active ingredient of the wound healing composition, of MOF-808 which is the MOF prepared in Example 1, the following experiment was conducted. For the experiment, a method suggested in the ISO 10993 biological evaluation of medical devices- Part 5: Test for *in vitro* cytotoxicity, test on extracts was used, and a cell line used herein was mouse fibroblasts (NIH 3T3, ATCC).

For cytotoxicity evaluation, the mouse fibroblasts were used after subculturing in DMEM (Gibco, DMEM, powder, high glucose) containing 10% fetal bovine serum (Gibco, Certified FBS) for 3 to 4 passages. The subcultured cells were seeded at 1×10⁵ cells/well in 96-well plates and cultured in DMEM by monolayer culture, the medium was removed, and the MOF-808 prepared in Example 1 was treated at 0.1, 1 or 10 µg/ml. Here, for a control, only DMEM was treated. Afterward, the fibroblasts of each sample were incubated in 5% CO₂ at 37 °C in an incubator for 24, 48 or 72 hours and observed under a microscope (OLYMPUS, CKX53 inverted microscope), and cell viability was measured by an MTT assay. The results are shown in FIG. 1.

As shown in FIG. 1, in the experiments performed for 24, 48 and 72 hours, the fibroblasts treated with 0.1 to 10 µg/ml of MOF-808 showed cell viability corresponding to the control. Specifically, the values of the cell viability at 0.1 and 1 µg/ml for 24, 48 and 72 hours were similar to or superior to that of the control. In addition, the values of the cell viability at 10 µg/ml for 48 and 72 hours were approximately 87.9% and 88.5% of the control. Particularly, compared to the control, it can be seen that the cell viability of the fibroblasts treated with 0.1 µg/mL or less of the MOF-808 was excellently maintained.

Accordingly, it can be seen that the MOF-containing composition according to one embodiment of the present invention has no significant difference in cytotoxicity, compared to the control. Since this value corresponds to Grade 1 (cell viability: 80% or more) satisfying the cytotoxicity standard of the standards for approving products of wound healing materials of the Korean Food and Drug Administration (in the evaluation by the cytotoxicity test (elution method) described in ISO 10993-5, Grade 2 is appropriate when 50% or more cells are viable, and there is less cell hemolysis and the empty space between cells is not wide), the MOF-containing composition according to one embodiment of the present invention has excellent safety when applied to a wound site.

### Experimental Example 2 Evaluation of wound healing ability of MOF (Migration assay)

To evaluate the wound healing ability of MOF-808, which is the MOF prepared in Example 1, an *in vitro* scratch wound assay was conducted as follows.

First, fibroblasts which are the same as the mouse fibroblasts used in Experimental Example 1 were seeded at 5×10⁶ cells/well in 6-well plates and cultured in DMEM (Gibco, DMEM, powder, high glucose) through monolayer culture. Scratches were generated at 100% confluence, followed by removing suspended cells. Subsequently, to confirm the effectiveness of the MOF-808 of Example 1, the MOF-808 was treated by being dispersed at 1, 10 and 100 ng/ml in serum-free DMEM, and then the cells were observed under a microscope (OLYMPUS, CKX53 inverted microscope) at a predetermined time (0, 5, 10 and 20 h), and an area was calculated using Image J (NIH). The results are shown in FIGS. 2A and 2B. Here, the control was treated with untreated serum-free DMEM.

As shown in FIGS. 2A and 2B, compared to the control, in the experimental groups treated with 1, 10 and 100 ng/ml of MOF-808, high wound healing ability was exhibited. This result is due to the presence or absence of MOF-808, and as a result of measuring the migration of fibroblasts over time, it was confirmed that, as time passes and the MOF-808 concentration increases, that is, in the experimental group treated with 100 ng/ml of MOF-808, the migration of fibroblasts was fast.

Therefore, the MOF-containing composition according to one embodiment of the present invention has excellent wound healing ability, which is the wound healing ability according to the promotion of fibroblast migration. Accordingly, the MOF-containing composition according to one embodiment of the present invention has an excellent wound healing effect.

### Experimental Example 3 Evaluation of wound healing ability in animal model treated with wound dressing material containing MOF

To evaluate wound healing ability and confirm the efficacy as a wound dressing material of the animal model of MOF-808, which is the MOF prepared in Example 1, the following animal test was conducted using the wound dressing material prepared in Example 2, that is, the MOF-808-containing wound dressing material. The animal test was conducted with 4-week-old female (240 to 270 g) Sprague-Dawley rats as animal models.

Specifically, for acclimation to a laboratory environment, rats were bred at 20 to 24 °C and a humidity of 40 to 60% in an environment in which light/dark is automatically controlled at 12-hour intervals for one week before the test. A control and an experimental group of dressing materials for animal tests were sealed in an aluminum pouch and sterilized with gamma rays (25 kGy) before dressing. The control was a hydrogel (0% MOF) not containing the MOF-808 prepared in Example 1, which is an active ingredient, and the experimental group was a hydrogel containing 0.2 wt% MOF-808 (0.2% MOF), which is the hydrogel prepared in Example 2, based on the total weight of the hydrogel. In the animal test, a round-shaped full-layer wound with a diameter of 12 mm was made on the skin of the dorsal area of a rat, each of the hydrogels of the control and the experimental group was uniformly applied to the wound site, the wound site was covered with a waterproof film, Tegaderm (3M, Tegaderm 1626W) for protection, and then the film was replaced every 4 days. The results are shown in FIGS. 3A and 3B. At this time, the wound healing process of a rat that was not dressed with either the hydrogel or MOF-808 on the wound site was also observed at the same time intervals, which corresponds to the control in FIGS. 3A and 3B.

As shown in FIGS. 3A and 3B, it can be seen that the full-layer wound on the skin of the dorsal area of the rat undergoes epithelialization as time passes after the treatment with the wound dressing material (hydrogel containing MOF-808) prepared in Example 2, resulting in wound healing. Compared with the control group, it was confirmed that the wound recovery rate of the experimental group was high enough to be visually observed.

Accordingly, it can be seen that the MOF-containing composition and wound dressing material according to one embodiment of the present invention have wound healing ability, and thus can be used as high-functional dressing materials for wound healing.

Therefore, the MOF according to one embodiment of the present invention has excellent wound healing ability, and a wound dressing material comprising a hydrophilic superabsorbent polymer in which the MOF is dispersed has excellent wound recovery ability and excellent wound healing ability.

## Claims

1. A composition for wound healing, comprising:
a metal-organic framework (MOF) as an active ingredient.

2. The composition of claim 1, wherein the MOF comprises one or more selected from the group consisting of Al and Zr, and one or more organic ligands selected from the group consisting of 4,4'-biphenyldicarboxylic acid, benzene-1,4-dicarboxylic acid, 9,10-anthracenedicarboxylic acid, biphenyl-3,3,5,5'-tetracarboxylic acid, biphenyl-3,4',5-tricarboxylic acid, 5-bromoisophthalic acid, 5-cyano-1,3-benzenedicarboxylic acid, 2,2-diamino-4,4'-stilbenedicarboxylic acid, 2,5-diaminoterephthalic acid, 1,1,2,2-tetra(4-carboxylphenyl)ethylene, 2,5-dihydroxyterephthalic acid, 2,2-dinitro-4,4-stilbenedicarboxylic acid, 5-ethynyl-1,3-benzenedicarboxylic acid, 2-hydroxyterephthalic acid, 2,6-naphthalenedicarboxylic acid, 1,2,4,5-tetrakis(4-carboxyphenyl)benzene, 4,4,4"-s-triazine-2,4,6-triyl-tribenzoic acid, 1,3,5-tricarboxybenzene, 1,4,7,10-tetraazacyclododecane-N,N',N",N'"-tetraacetic acid, 1,3,5-tris(4-carboxy1,1'-niphenyl- 4-yl)benzene, 1,3,5-tris(4-carboxyphenyl)benzene, and 1,3,5-triscarboxyphenylethynylbenzene.

3. The composition of claim 1, wherein the MOF is Zr₆O₄(OH)₄(BTC)₂(HCOO)₆ (MOF-808).

4. The composition of claim 1, wherein the MOF promotes the migration of skin cells.

5. The composition of claim 1, wherein the MOF is contained at 0.001 to 10 µg/ml.

6. The composition of claim 1, wherein the MOF is contained at 0.01 to 10wt% based on the total weight of the composition.

7. The composition of claim 1, wherein the wound is one or more selected from the group consisting of an injury, a non-healing traumatic wound, destruction of tissue by irradiation, an abrasion, a laceration, an avulsion, a penetrating injury, a bullet wound, a cut, a burn, frostbite, a sore, dry skin, keratoderma, a crack, chapped skin, dermatitis, a surgical or vascular disease wound, a bruise, a corneal wound, a bedsore, an abscess, a chronic ulcer, an operative suture site, a spinal injury, a gynecological wound, a chemical wound and an acne.

8. The composition of claim 1, wherein the composition is a composition for external application to the skin.

9. The composition of claim 1, wherein the composition is a pharmaceutical composition.

10. The composition of claim 1, wherein the composition is a cosmetic material composition.

11. The composition of claim 1, wherein the composition is a quasi-drug composition.

12. A wound dressing material comprising a hydrophilic superabsorbent polymer and a metal-organic framework (MOF).

13. The material of claim 12, wherein the MOF comprises one or more selected from the group consisting of Al and Zr, and one or more organic ligands selected from the group consisting of 4,4'-biphenyldicarboxylic acid, benzene-1,4-dicarboxylic acid, 9,10-anthracenedicarboxylic acid, biphenyl-3,3,5,5'-tetracarboxylic acid, biphenyl-3,4',5-tricarboxylic acid, 5-bromoisophthalic acid, 5-cyano-1,3-benzenedicarboxylic acid, 2,2-diamino-4,4'-stilbenedicarboxylic acid, 2,5-diaminoterephthalic acid, 1,1,2,2-tetra(4-carboxylphenyl)ethylene, 2,5-dihydroxyterephthalic acid, 2,2-dinitro-4,4-stilbenedicarboxylic acid, 5-ethynyl-1,3-benzenedicarboxylic acid, 2-hydroxyterephthalic acid, 2,6-naphthalenedicarboxylic acid, 1,2,4,5-tetrakis(4-carboxyphenyl)benzene, 4,4,4"-s-triazine-2,4,6-triyl-tribenzoic acid, 1,3,5-tricarboxybenzene, 1,4,7,10-tetraazacyclododecane-N,N',N",N'"-tetraacetic acid, 1,3,5-tris(4-carboxy1,1'-niphenyl- 4-yl)benzene, 1,3,5-tris(4-carboxyphenyl)benzene, and 1,3,5-triscarboxyphenylethynylbenzene.

14. The material of claim 12, wherein the MOF is Zr₆O₄(OH)₄(BTC)₂(HCOO)₆ (MOF-808).

15. The material of claim 12, wherein the hydrophilic superabsorbent polymer is a hydrogel.

16. The material of claim 12, wherein the MOF is contained at 0.01 to 10wt% based on the total weight of the hydrophilic superabsorbent polymer.
